# EUROPEAN PATENT SPECIFICATION

(11) **EP 1 724 260 B1**
(45) Date of publication and mention of the grant of the patent: **20.02.2008**
(21) Application number: 05076060.2
(22) Date of filing: 06.05.2005
(51) Int. Cl.: C07D 209/42

(54) **Process for the synthesis of (2S, 3aR, 7aS)octahydroindole-2-carboxylic acid and its conversion to trandolapril**
Verfahren zur Herstellung von ¬(2S, 3aR, 7aS)-octahydroindole-2-Carbonsäure und Umwandlung zum Trandolapril
Procédé de synthèse de l'acide (2S, 3aR, 7aS) octahydroindole-2-carboxylique et conversion en trandolapril

(43) Date of publication of application: 22.11.2006
(73) Proprietor: Sochinaz SA, 1895 Vionnaz (CH)
(72) Inventor: Akhtar, Haider, 1024-Ecublens (CH); Ramesh, Babu Potluri, Ameerpet, Hyderabad-500 038 (IN); Venkata, Subhramanian Hariharakrishnan, Hyderabad -500 018, Andhra Pradesh (IN); Hari Prassad Kodali, Hyderabad (IN)
(74) Representative: Burtin, Jean-François

(56) References cited:
- US-A- 4 933 361

## Description

### BACKGROUND OF THE INVENTION: The present invention relates to a new process for industrial synthesis of Trandol6pril.

This invention more precisely relates to the synthesis of organic compounds related to L-alanine, which are starting materials for synthesizing building blocks needed for the production of indole-like inhibitors of Angiotensin I Converting Enzyme (IACE), namely Trandolapril and its derivatives.

More specifically the invention relates to a new synthesis of Trandolapril and other indole-like IACE, which are potent hypertension inhibitors.

Trandolapril is a known antihypertensive agent defined as (2S, 3aR, 7aS)-1-[(1S)-1-ethoxycarbonyl)-3-phenylpropylamino-1-oxopropyl] octahydro-[1 H]-indole-2-carboxylic acid. Trandolapril has the following structural formula:

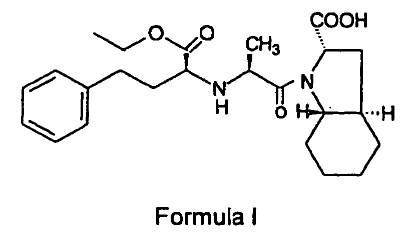

The general approach in most of the Trandolapril synthesis is a peptide coupling of N-[(1-ethoxy carbonyl)-3-phenyl propyl)-S-alanine with benzyl-(2s,3aR,7aS)-octahydroindole-2-carboxylate using as coupling agent dicyclohexylcarbodiimiide in combination with 1-hydroxy benzotriazole or n-alkyl phosphonic anhydride in presence of an organic base, such as triethylamine. (2S,3aR,7aS)-octahydroindole-2-carboxylic acid is a key intermediate for the synthesis of trandolapril, which is described in the US Patent 4,525,803.

The synthesis of the key intermediate is described in the following patents or publications viz., Tetrahedron Letters, Vol. 24, (48), 5339-5345; Tetrahedron Letters, Vol. 24, (48), 5347-5350 ; US Patent 4.879.392; US Patent 49633361 / EP 084164 ; Tetrahedron Letters Vol. 35 (54), 4889-4892; and US Patent 6, 559, 318.

The synthesis of octahydroindole-2-carboxylic acid as described in Tetrahedron Letters, Vol. 24, (48), 5339-5345 is given in the scheme-I

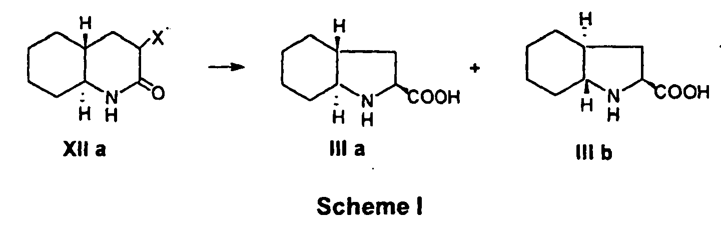

In this method, trans decahydroquinoline derivative of formula-Xlla is subject to Favorskii type ring contraction, followed by hydrolysis to give a mixture of III a and III b as a 1:1 mixture.

A similar reaction with cis derivative XII b gives a mixture of IIIc and IIId as a 9:1 mixture.

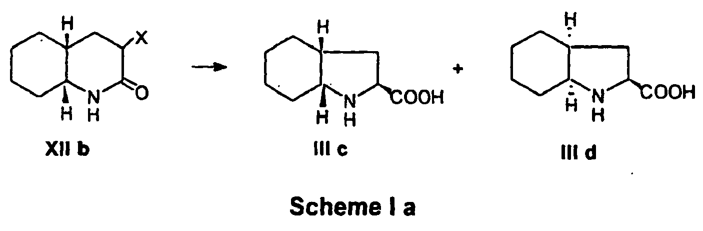

The selectivity for IIIc over IIId, when the reaction is conducted with cis lactam Xllb, is due to less thermal instability of IIIb on account of 1,3-cis interaction of a carboxyl group and a six-member ring. Such interaction, is not present in IIIa and IIIb, formed from trans lactam XIIa, hence the product is formed as a 1:1 mixture
The scheme-II describes the methodology used in Tetrahedron Letters, Vol. 24, (48), 5347-5350 for the preparation of trans octahydroindole-2-carboxylic acid 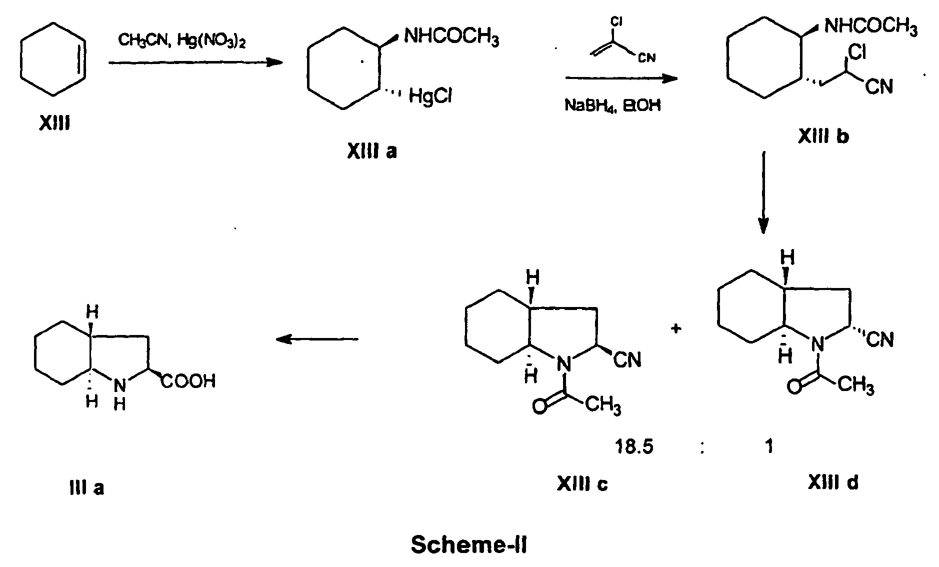 Reaction of cyclohexene with acetonitrile and mercuric acetate followed by ligand exchange with sodium chloride gives the crystalline acetamidomercury chloride in 98% yield. Reaction of the product of formula XIIIa with α-chloro acrylonitrile followed by reaction with NaBH4 and ethanol gives the product of formula XIIIb, which is cyclized with sodium in DMF to get a mixture of Xlllc and XIIId in the ratio of 18.5 : 1. On hydrolysis, IIIa is obtained selectively.

Another method of preparation for octahydroindole-2-carboxylic acid is disclosed in the US Patent 4,879,392, and is reported in scheme III

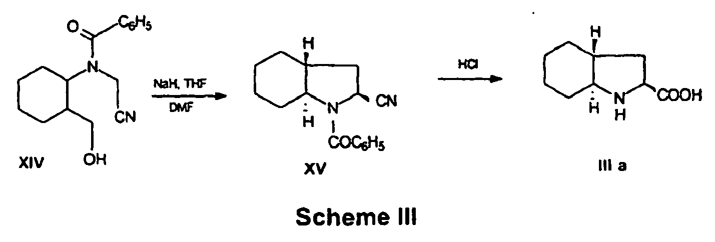

Herein, the cyclohexane derivative of formula XIV is converted into octahydroindole-2-carbonitrile the derivative of formula XV, which is hydrolyzed to give octahydroindole-2-carboxylic acid of formula III a.

Another method for the synthesis of octahydroindole-2-carboxylic acid and its subsequent conversion to trandolapril is disclosed in the US Patent 4933361/EP 084164 and given in the scheme IV

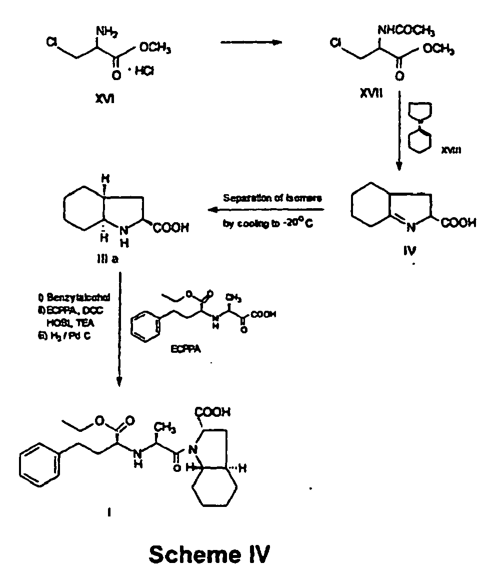

In this patent, methyl-β-chloro alaninate hydrochloride of formula XVI is acetylated to give a product of formula XVII, which is treated with the enamine derivative of formula XVIII to give hexahydroindoline-2-carboxylicacid of formula-IV. The product of formula IV is hydrogenated and the required enantiomer is isolated by cooling to -20°C. (2S,3aR,7aS)-Octahydroindole-2-carboxylic acid is first esterified with benzyl alcohol, coupled with ECPPA using DCC/HoBT, and finally debenzylated to yield trandolapril.
Tetrahedron Letters Vol. 35 (54), 4889-4892 describes another methodology for the synthesis of (2S,3aR,7aS)-octahydroindole-2-carboxylic acid, which is depicted in scheme V

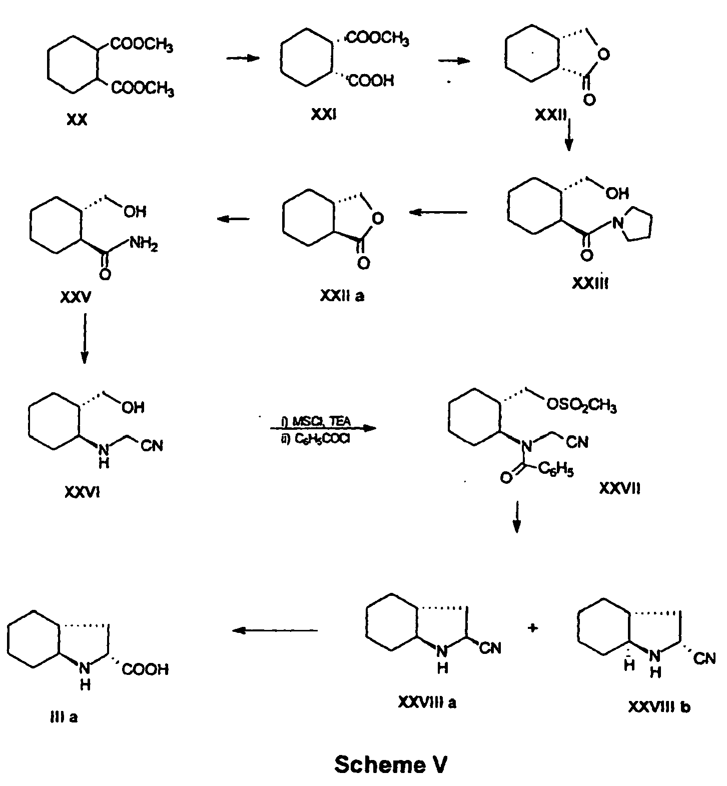

Dimethyl-1,2-cyclohexane dicarboxylate of formula XX is enzymatically resolved to give the monomethyl ester of 1,2-cyclohexane dicarboxylic acid of formula XXI, which is converted into hexahydroisobenzofuranone of formula XXII. The product of formula XXII is reacted with pyrrolidine to yield a product of formula XXIII which is converted to hexahydroisobenzofuranone of formula XXII a. This product is treated with ammonia to give cyclohexane carboxamide of formula XXV. This product is subject to the Hoffmann reaction, followed by reaction with formaldehyde and potassium cyanide to give cyclohexyl amine derivative of formula XXVI. The product of formula XXVI, in reaction with methane sulphonyl chloride and benzoyl chloride give a product of formula XXVII. This product is converted into a mixture of octahydroindole-2-carbonitrile of formula XXVIII a and XXVIII b. Octahydrindole-2-carbonitrile is hydrolyzed to give octahydroindole-2-carboxylic acid of formula III a.
The process for the synthesis of (2S,3aR,7aS)-octahydroindole-2-carboxylic acid is described in the US Patent 6,559,318 and reported in the scheme VI. 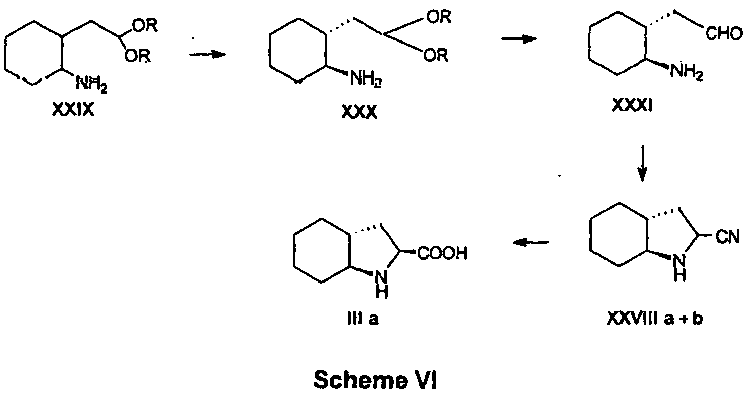 In this method, cyclohexylamine derivative of formula-XXIX is resolved to produce enantiomerically pure product of formula XXX, which is converted to octahydroindole-2-carbonitrile of formula XXVIII a. The product of formula XXVIII a on hydrolysis yields the octahydroindole-2-carboxylic of formula III a.

The above description gives various methods adopted to synthesize octahydroindole-2-carboxylic acid, which is the key intermediate in the preparation of trandolapril. After analyzing the different methods, it can be concluded that except the methodologies described in the US Patent 4963361/EP 084164, all the other methods are not suitable for industrial purpose.

The method described in the US Patent 4933361/EP 084164 has also the following drawbacks:
i) The synthesis of methyl -β-chloro alaninate makes use of phosphorous pentachloride, which is a corrosive reagent and difficult to handle.
ii) Isolation of (2S,3aR,7aS)-octahydroindole-2-carboxylic acid at -20°C is a difficult attempt during the scale up
iii) Use of dicyclohexylcarbodiimiide in combination with hydroxybenzotriazole makes the process costlier

Considering all these aspects, it appears that there is a need to develop an alternative, eco-friendly and industrially viable process.

### SUMMARY OF THE INVENTION

One of the objectives of the present invention is to develop an economical method for the preparation of the key intermediate viz, (2S,3aR,7aS)-octahydroindole-2-carboxylic acid. Another objective of the invention is to develop an economical method for converting the key intermediate into trandolapril.

Yet another objective of the invention is to develop a methodology for the preparation of trandolapril by an eco-friendly process, which is industrially and economically viable.

After a thorough analysis of the various methods for the synthesis of the crucial intermediate viz, (2S,3aR,7aS)-octahydroindole-2-carboxylic acid, it was concluded that the best starting material is L-serine. US Patent 4963361/EP 084164, utilizes the methyl -β-chloro alaninate hydrochloride, which is obtained from L-serine methyl ester hydrochloride. The major bottleneck is the conversion of L-serine methyl hydrochloride to methyl -β-chloro alaninate hydrochloride, which involves the use of phosphorus pentachloride. Phosphorus pentachloride is a corrosive reagent and difficult to handle. The above mentioned conversion involves the addition of phosphorus pentachloride in installments and this is a problematic process.

One of the objectives of the present invention is to replace phosphorus pentachloride, which is a corrosive reagent. Another objective is to use a novel intermediate for the synthesis of octahydroindole-2-carboxylic acid. The best way was thought in replacing a chloro substituent with another good leaving group. This can be achieved using an acyloxy group ie. by converting L-serine methyl ester hydrochloride to N-acetyl-β-acyloxy alanine ester of formula II.

The objective of synthesizing octahydroindole-2-carboxylic acid by an economical and eco-friendly process using a novel intermediate was achieved by reacting N-acetyl-β-acyloxy alanine ester of formula II 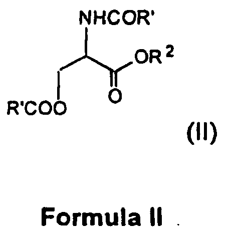 wherein R' is a lower alkyl radical such methyl, ethyl, propyl etc. and R2 is a lower alkyl radical such as methyl, ethyl etc. with an enamine of formula IV 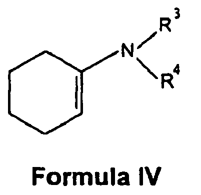 wherein R3 and R4 are each an alkyl group or together with nitrogen atom to which they are linked, a cyclic structure, which may contains an heteroatom selected from the group consisting of oxygen, sulphur or imino to give a cyclohexanone derivative of formula-V 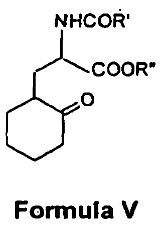 which is hydrolytically cyclized to give hexahydroindole-2-carboxylic acid of formula-VI 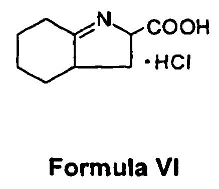 which on further hydrogenation gives octahydroindole-2-carboxylic acid hydrochloride of formula VII 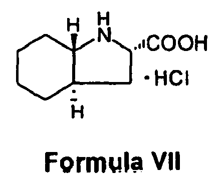 wherein the hydrogens in the position 2, 3a, and 7a have the configuration S,R & S respectively.

The product of formula-VII is then esterified by a benzyl alcohol of the formula VIII a 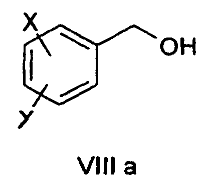 in the presence of an aryl sulphonic acid to produce an aryl sulphonic acid salt of a benzyl ester of formula-VIII, 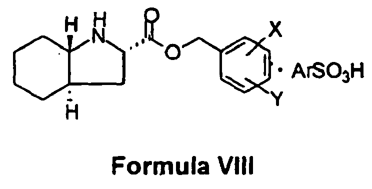 wherein Ar is an aromatic ring having up to 10 carbon atoms
wherein X and Y are an hydrogen atom, an halogen, alkyl or alkoxyl group and Ar is an aryl group,
which is further converted into a halo hydric acid addition salt of formula-IX

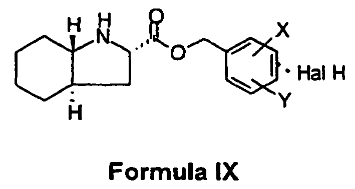

The product of formula IX is then neutralized and resolved with an optically - active acid agent such as diaroyl-L-tartaric acid, L-mandelic acid, d-10-camphor sulphonic acid, N-benzoyloxycarbonyl-L-phenylalanine to give a salt of formula-X 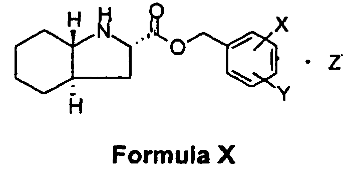 where Z is an optically active acid such as diaroyl-L-tartaric acid, L-mandelic acid, d-10-camphor sulphonic acid, N-benzoyloxycarbonyl-L-phenylalanine and the like.

The product of formula-X is neutralized and coupled with the compound of formula-XI 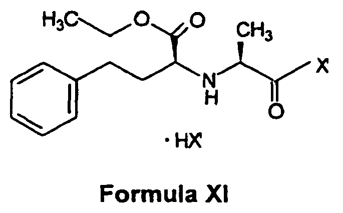 to give a product of formula XII

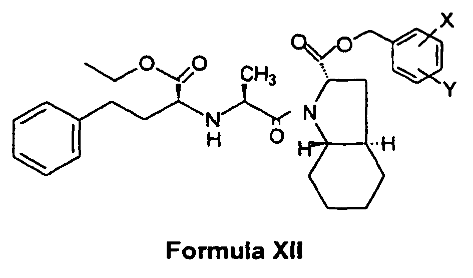

The benzyl ester of formula-XII is then debenzylated by hydrogenolysis in the presence of a catalyst to yield trandolapril of.formula-1, the latter is further purified by crystallization from an organic solvent.

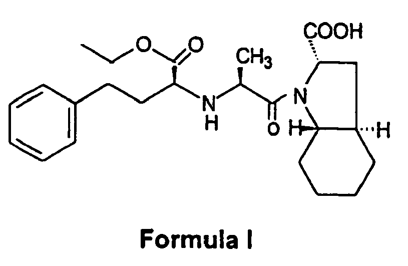

In order to study the nature of the dipeptide formed from the unresolved benzyl octahydroindole-2-carboxylate, the product of formula IX is neutralized and coupled with ECPPA derivative of formula XI to give benzyl trandolapril. The latter is debenzylated to give trandolapril, which on crystallization from suitable solvents yields the desired diastereoisomer product, which is identical in all respects to the product obtained from enantiomerically pure benzyl octahydroindole-2-carboxylate.
The choice of the reaction conditions and a judicious choice of solvents employed to obtain the desired diastereoisomer in yields which are highly advantageous on an industrial scale. Furthermore, the synthesis according to the process of the invention offers the advantage of employing inexpensive coupling reagent, which is of importance on an industrial scale.

In a preferred embodiment, the process of this invention is further defined by the following features:
- The acylating agent is preferably a functional derivative of an alkyl carboxylic acid such as an acid anhydride for example acetic anhydride, propionic anhydride or butyric anhydride, to produce a O.N-diacylated-L- serine derivative in an inert solvent such as toluene or xylene,
- The secondary amine which is condensed with cyclohexanone to produce the enamine of formula IV, is pyrrolidine, piperidine, morpholine, azepine, homoazepine and thiadiazine,
- The formation of the enamine of formula IV is achieved in an inert solvent under dehydrating conditions,
- The enamine IV may also be formed during the course of the reaction without isolation,
- The reaction of the acylated derivative of formula II with the enamine of formula IV is achieved in a non-polar solvent, in the presence of an organic base, to produce the 2-oxocyclohexyl alaninate of formula IV,
- The reaction of the acylated derivative of formula II with the enamine of formula IV is moreover achieved at a temperature ranging from 0 to 150°C
- The 2-oxo cyclohexyl derivative of formula V is cyclized into indole carboxylic acid of formula VI by means of an acidic reagent, namely hydrochloric acid or sulphuric acid at reflux temperature for 2 to 6 hours, into an hexahydroindole-2-carboxylic acid derivative of formula VI.
- The hexahydroindole-2-carboxylic acid derivative VI is hydrogenated into an octahydroindole-2-carboxylic derivative of formula VII in the presence of a catalyst selected from the group consisting of Pd, Pt, Rh and the oxides thereof, in an acidic medium, like acetic acid, or propionic acid, at a temperature ranging from 15 to 50°C and at a pressure of 10 to 60 psi.
- The esterification of the octahydroindole-2-carboxylic acid with a benzyl alcohol is performed in the presence of an arylsulphonic acid such as p. toluene sulphonic acid or naphthalene sulphonic acid to produce the arylsulphonic acid addition salt of the benzyl ester of the acid of formula VIII
- The arylsulphonic acid addition salt of formula VIII is further converted into a halohydric acid addition salt by using an alcoholic solution of an halohydric acid.
- The resolution of the hydrochloride of the benzylester of formula IX is achieved using a diester of L-tartaric acid or L-mandelic acid or d-camphosulphonic acid, to obtain a salt of formula X.
- The diester of L-tartaric acid is a benzoyl-, a naphtoyl-, a vanilloyl- or a dichlorobenzoyl ester of L-Tartaric acid.
- The coupling of the compound of formula X with ECPPA acid functional derivative of formula-XI as the hydrochloride is achieved using a trialkylamine and an halohydric acid salt of a functional derivative of ECPPA such as the acid chloride derivative.
- The conversion of the compound of formula XII into trandolapril (I) is achieved by hydrogenolysis using hydrogen in the presence of a catalyst selected from the group consisting of Pd/C, Rh/C, and Pt/C, and the like.
- The purification of the thus obtained trandolapril is achieved by crystallisation from a low-boiling solvent such as alkanol and alkyl ether.
- The product of formula IX is neutralized and coupled with a product of formula XI
- The benzyl dipeptide thus obtained is debenzylated using hydrogen in the presence of a catalyst selected from the group consisting of Pd/C, Rh/C, and Pt/C, and the like.
- The purification of the crude product is achieved by crystallization from a low boiling solvent such as alkanol and alkyl ether.

### DETAILED DESCRIPTION OF THE INVENTION

The object of the invention was to use a novel intermediate in the preparation of octahydroindole-2-carboxylic acid. Another objective was to use an economical method for resolving the octahydroindole-2-carboxylic acid. Yet another objective was to couple the enantiomeric bicyclic acid with ECPPA by a newer methodology. Hence it was decided to study the reaction of L-serine with an alkanoic acid anhydride like acetic anhydride, propionoic anhydride, butyric anhydride etc. The reaction was preferably conducted utilizing excess of the alkanoic acid anhydride at 0°C to 150 °C. The reaction was more preferably conducted using 2 to 3 moles excess of alkanoic acid anhydride in the presence of an organic solvent like toluene, xylenes etc., at 0 to 150°C. The reaction of L-serine with alkanoic acid anhydride was most preferably conducted in an organic solvent like toluene, xylenes etc, at 80 to 150°C. The reaction was preferably conducted for 8 to 12 hours and then concentrated. The residue was cooled to about 25°C and dissolved in an organic solvent like aromatic hydrocarbons viz., toluene, xylenes etc. or more polar solvents like diethyl ether, diisopropyl ether, tetrahydrofuran, 1,4-dioxane etc. or aprotic dipolar solvent like, acetone, acetonitrile, N,N-dimethylformamide, N,N-dimethylacetamide etc or alkanols like methanol, ethanol, propanols etc.

The solution of methyl N-acyl-β-acyloxy alaninate in an organic solvent mentioned herein was treated with an enamine of formula IV at a temperature of 0°C to 150°C in the presence of an organic base. The enamine was prepared by known art and was preferably reacted with methyl-N- acyl-β-acyloxy alaninate in an organic solvent in the presence of an organic base like triethyl amine, diisopropyl ethylamine, N-methyl morpholine etc., at a temperature of 10 to 100°C for a duration of 8 to 10 hours. The progress of the reaction was monitored by thin layer chromatography. On completion, solvent was distilled off under reduced pressure, the residue was treated with 2N acid like hydrochloric acid, sulphuric acid etc and extracted with an organic solvent like dichloromethane, dichloroethane, chloroform, ethyl acetate, butylacetate etc. The organic extract was concentrated under reduced pressure and used for the next stage without any further purification

The residue obtained was treated with an acid like dilute hydrochloric acid or sulphuric acid, at a temperature of 50 to 150°C to effect hydrolytic cyclization. The hydrolytic cyclization was preferably conducted using 4 to 6N hydrochloric acid or sulphuric acid at a temperature of 85 to 110°C for a duration of 4-8 hours. The progress of the reaction was monitored by thin layer chromatography. On completion, the aqueous solution was concentrated under reduced pressure. The residue was dissolved in an alkanol like methanol, ethanol or isopropanol containing hydrogen chloride and treated with a co-solvent like diethylether, diisopropylether, acetone, methyethylketone, acetonitrile etc to precipitate the product of formula-VI and was characterized by spectral analysis.

The product of formula VI viz, hexahydroindole-2-carboxylic acid hydrochloride was, hydrogenated in a solvent like acetic acid, propionic acid etc using a catalyst selected from the group consisted of palladium, platinum, rhodium, and their oxides etc. The hydrogenation was preferably conducted in alkanoic acid at a temperature of 25 to 60°C. After completion of the hydrogen take up, solvent was distilled off at reduced pressure, the residue was dissolved in an alkanol like methanol, ethanol, isopropanol containing hydrogen chloride and the product of formula-VII was precipitated by adding a co-solvent like diethyl ether, diisopropylether, acetone, acetonitrile etc. The product was characterized by spectral analysis.

The product of formula-VII was then esterified with benzyl alcohol using aryl sulphonic acid in an aromatic hydrocarbon solvent like toluene, xylenes etc to give the product of formula-Vill. The mole ratio of the substrate benzyl alcohol / aryl sulphonic aicd was preferably 1 / 1 to2 / 1.5 to 2 and the reaction was preferably conducted at 80°C to 120 °C using a Dean-Stark apparatus. The product of formula-VII was converted to the product of formula-Vili by the method described herein and after completion of the esterification was processed and converted to product of formula-IX.

The next objective was the preparation of enantiomerically pure benzyl (2S,3aR,7aS)-octahydroindole-2-carboxylate. This was achieved by using a resolving agent like diaroyl-L-tartaric acid or 10-d-camphor sulphonic acid or L-mandelic acid or benzoyloxycarbonyl-L-phenylalanine. The process was preferably carried out by treating the product of formula IX with a base, extracting the neutralised bicyclic acid ester and treating the ester with the resolving agent. Benzyl octahydroindole-2-carboxylate was preferably treated with 0.6 to 1.2 equivalent of the resolving agent in a solvent mixture comprising of methyl/ethyl/isopropyl/ acetate and dimethyl/diethyl/diisopropyl/tertiary, butyl methyl ether, tetrahydrofuran/1,4 dioxane. The salt of benzyl (2S,3aR,7aS)-octahydroindole-2-carboxylate with the resolving agent was preferably separated from any of the solvent system mentioned earlier at a temperature of 0°C to 35°C. It was more preferably separated at a temperature of 15°C to 25°C. The separated salt was neutralized with a base, like sodium/potassium hydroxide, sodium/potassium carbonate/bicarbonate and extracted into an organic solvent like dichloromethane, diisopropylether ethylacetate etc. The solution was concentrated and treated with an alkanol containing hydrogen chloride to convert into enantiomerically pure benzyl-(2S,3aR,7S)-octahydroindole-2-carboxylate hydrochloride which showed an optical rotation of-48.6° (c = 1 in methanol)

The third objective of utilizing an alternate coupling to form dipeptide was achieved by conducting the reaction of benzyl (2S,3aR,7aS)-octahydroindole-2-carboxylate with ECPPA acid chloride hydrochloride. The use of costly reagents like dicyclohexylcarbodiimide in combination of hydroxybenzotriazole was avoided. The preparation of ECPPA acid chloride hydrochloride is very easy and using this intermediate for coupling makes the process very economical. The enantiomerically pure benzyl (2S,3aR,7aS)-octahydroindole-2-carboxylate was coupled with ECPPA acid chloride hydrochloride preferably in a solvent dichloromethane, ethylacetate, isopropylacetate in the presence of a base like triethyl amine, diisopropylethyl amine or N-methylmorpholine at 0°C ro 25 °C. The reaction was conducted more preferably at 10 °C to 15°C for 10 to 15 hours. The progress of the reaction was monitored by thin layer chromatography and on completion was worked up to give the product of formula XII.

The product of formula XII was debenzylated in an alkanol at 10 °C to 30 °C using Pd/C, Rh/C and Pt/C as catalyst. After the hydrogenation, the product viz trandolapril was isolated and purified by crystallization. The crystallization was preferably performed in a solvent comprising of an alkanol and an ether to give trandolapril in a good yield and an excellent purity.

The synthesis of the dipeptide by using the racemic benzyl octahydroindole-2-carboxylate was achieved and the resulting product was compared with the product obtained from the enantiometrically pure benzyl octahydroindole-2-carboxylate.

The product of formula IX was neutralized and coupled with ECPPA using DCC/HoBT or with ECPPA derivative of formula-XI, wherein X=CI using a base like trialkyl amine to give the benzyl dipeptide, which was debenzylated using a catalyst like Pt/C, Pd/C or Rh/C and like. The product obtained was crystallized from the alkanol like methanol, ethanol, isopropanol containing an ether like diethylether, diisopropylether, tertiary butyl methyl ether, tetrahydrofuran etc. The product was compared with the one obtained from the resolved benzyl octahydroindole-2-carboxylate and ECPPA derivative. The physico-chemical proprieties of both products were identical, in the parameters such as the melting point, the specific optical rotation, [¹H] NMR, [¹³C] NMR etc.
This preparation using unresolved benzyl octahydroindole-2-carboxylate is highly economical.

The advantages achieved by this invention are
i) preparation of octahydroindo-2-carboxylic acid by utilizing methyl-N-acetyl-β-acyloxy alaninate, which makes the process economical and eco-friendly
ii) resolution by a cheap resolving reagent like diaroyl-L-tartaric acid/10-d-camphor sulfonic acid/ L-mandelic acid/benzoyloxycarbonyl-L-phenylalanine.
iii) forming the dipeptide by coupling the bicyclic acid ester with ECPPA acid chloride hydrochloride, which makes the process more economical and yields trandolapril in good yield and high purity
iv) preparation of trandolapril by using the unresolved benzyl octahydroindole-2-carboxylate and ECPPA derivative is a highly economical process
The following examples illustrate the present invention in more detail, but are not to be construed to limit its scope in any manner. Melting points are uncorrected.

### Examples :

### Example-1

### Preparation of ethyl-β-acyloxy-N-acyl-s-alaninate:

In a three necked one-liter flask fitted with a mechanical stirrer, condenser and an addition funnel was added toluene 500ml and methyl-β-hydroxy-s-alaninate hydrochloride 100gms. Acetic anhydride 400gms was added from the addition funnel at 25 °C to 30 °C. After the addition the reaction mixture was slowly heated to about 70 °C to 80 °C and was maintained at that temperature for 3 hours. After the maintenance, totuene was distilled off under reduced pressure. Additional quantity of toluene (300mt) was added and the distillation process was continued.

The residue obtained, was dissolved in diethyl ether. The ethereal solution was diluted with hexane. On cooling a colourless solid precipitated out, which was filtered, washed with a mixture of diethyl ether and hexane and dried.

| | | | | |
|---|---|---|---|---|
| M.P : | 74-76°C | | | |
| I R : | 3276, 1744, 1645, 984, 829 cm⁻¹ | | | |
| NMR : | (CDCl₃,δ): | 2.06 (S, 6H, 2 X CH₃) | | |
| | | 3.79 (S, 3H, OCH₃) | | |
| | | 4.87 (M, 1H, CH) | | |
| | | 6.31 (6d, 1H, NH) | | |
| Mass : | 204 (28%, M+1) | | 162 (10%) | 144 (100%) |
| [α]_{D}²⁵ (C=1, DMF): | + 12.2° | | | |

### Example-2

### Preparation of hexahydro[1H]indole-2-carboxytic acid hydrochlorde

a) In a three necked 1 It RB flask fitted with a mechanical stirrer, condenser and an addition funnel was added toluene 500ml and methyl β-hydroxy-s-alaninate hydrochloride (L-serine methylester hydrochloride) 100gm was added. To the stirred reaction mixture was added acetic anhydride 300ml from the addition funnel. After the addition, the reaction mixture was heated to about 70°C to 80 °C under stirring and maintained at that temperature for about 3 hours. Subsequently toluene was distilled off under reduced pressure. The residue was taken in fresh toluene and used in stage C
b) In a three-necked 1 It RB flask fitted with a mechanical stirrer, condenser and an addition funnel was added toluene 300ml, pyrrolidine 50gm and cyclohexanone 60gm. The mixture was fitted with Dean and Stark apparatus. The reaction mixture was stirred under reflux and water was removed azeotropically. When the water collection had ceased, the reaction mixture was cooled and used in stage C.
c) The solution obtained under (a) was taken in a 2 It three-necked RB flask fitted with a mechanical stirrer, condenser carrying a guard tube and an addition funnel. The solution obtained under (b) was taken in the addition funnel and added drop-wise to the stirred reaction mixture at 25 °C to 30 °C. After the addition, the reaction mixture was stirred at 25 °C to 30 °C for 12 hours to 14 hours. At the end of the maintenance, toluene was distilled off under reduced pressure, the residue was treated with 400ml of water, the pH was adjusted to 2 with hydrochloric acid and the reaction mixture was stirred for 15min. The reaction mixture was extracted with ethyl acetate (3 X 250ml), the ethyl acetate extract was washed with water and ethyl acetate was distilled off under reduced pressure
   The residue obtained above was treated with 4N hydrochloric acid 500ml and taken in a 1 It RB flask fitted with a stirrer, and condenser. The reaction mixture was stirred at reflux for 4 hours and then concentrated under reduced pressure.
   The residue obtained, was dissolved in methanolic hydrochloride. The methanolic solution was diluted with 500ml acetone. On cooling a colourless solid precipitated out, which was filtered, washed with a mixture of methanol and acetone and dried
   Yield = 118gms (80.55%)
   Melting point: 179-182 °C

### Example-3

### Preparation of octahydroindole-2-carboxylic acid hydrochloride

The product obtained in example-2 was dissolved in acetic acid 600ml and charged into a Parr hydrogenator. A slurry of 10% palladium charcoal (3gms) in acetic acid was also charged into the hydrogenator. Hydrogenation was carried out at 40 °C to 50 °C under 40 psi pressure and when the hydrogen take up had ceased, the system was cooled and the reaction mixture was filtered to remove the catalyst. The acetic acid solution was concentrated under reduced pressure. The residue obtained was dissolved in 100m1 of isopropyl alcohol and cooled to 0 °C. The obtained crystals were filtered and dried.
Yield = 104 gms
Melting point: 174-175 °C

### Example-4

### Preparation of phenyl methyl octahydroindole-2-carboxylate hydrochloride

Octahydroindole-2-carboxylic acid hydrochloride 50gms was dissolved in 300ml of toluene and 65gm of benzyl alcohol. 85gm of para toluenesulfonic acid was added and azeotropically water was removed (around 3 hours) and 200ml of water was added. The mass pH was adjusted to 11 with sodium hydroxide and the organic layer was separated. The organic layer was concentrated under reduced pressure. The residue obtained, was dissolved in methanolic hydrochloride 100ml. The methanolic solution was diluted with 500ml acetone. On cooling a colourless solid precipitated out, which was filtered, washed with a mixture of methanol and acetone and dried.
Yield = 63 gms
Melting point: 124-126 °C

### Example-5

### Preparation of phenylmethyl-(2S,3aR,7aS)-octahydroindole-2-carboxylate hydrochloride

Phenyl methyl octahydroindole-2-carboxylate hydrochloride 50gm was taken in 500ml of water and 10gm of sodium hydroxide was added. The mass was stirred for one hour and 300ml of dichloromethane was added. The bottom organic layer was separated and aqueous layer discarded. The solvent was removed under vacuum and the residue was dissolved in 50ml of methanol. 94gm of O,O'-dibenzoyl-L-tartaric acid was dissolved in 50ml of methanol and added to the substrate. After an hour the mixture was diluted with 300ml diisopropylether. The obtained crystals were filtered and the wet cake was taken in 200ml water. The pH was adjusted to 9-10 with sodium hydroxide after adding 250ml of dichloromethane. The organic phase was separated and aqueous phase was extracted with another 100ml of dichloromethane. The combined dichloromethane layer washed with 100ml of water and dried with sodium sulphate. The solvent was removed under vacuum and the residue was dissolved in 50ml of methanolic hydrochloride. The methanolic solution was diluted with 500ml acetone. On cooling a colourless solid precipitated out, which was filtered, washed with a mixture of methanol and acetone and dried.
Yield = 22 gms
Melting point = 152-154 °C
Specific optical rotation = - 48.6° (C = 1 in MeOH)

### Example-6

### Preparation of Trandolapril

Phenyl methyl-(2S,3aR,7aS)-octahydroindole-2-carboxylate hydrochloride 50gms was taken in 500ml of water and 10gms of sodium hydroxide was added. The mass was stirred for one hour and 300ml of dichloromethane was added. The bottom organic layer was separated and the aqueous layer discarded. The organic layer was dried over sodium sulphate. The dried organic layer was taken in a flask and ECPPA acid chloride hydrochloride, and triethylamine was added at room temperature. The mass was stirred for 3hours at room temperature. The inorganic salt was filtered and the filtrate was concentrated under reduced pressure. The filtrate was concentrated in vacuo, the residue was taken up in 200ml of ethanol and 6.5 g of Pd/C (10% of Pd) were added-hydrogenation was carried out less than 2 bar. After filtration, the mixture was concentrated and the residue was taken in diethyl ether. The obtained crystals were filtered and dried to give trandolapril-
Yield = 52 gms
Melting point = 124-126 °C
Specific optical rotation = - 17.9° (C = 2 in ethanol)

### Example 7

### Preparation of Trandolapril (alternate method)

Phenyl methyl-(2S,3aR,7aS)-octahydroindole-2-carboxylate hydrochloride 50gm was taken in 500ml of water and 10gms of sodium hydroxide was added. The mass was stirred for one hour and 300ml of dichloromethane was added. The bottom organic layer was separated and aqueous layer discarded. The organic layer was dried over sodium sulphate. The dried organic layer was taken in a flask and ECPPA, hydroxybenzotriazole, triethylamine were added at room temperature. After 15min dicyclohexyl carbodiimide was added and stirred for 20hours. The inorganic salt was filtered and the filtrate was concentrated under reduced pressure. The filtrate was concentrated in vacuo, the residue was taken up in 200ml of ethanol and 6.5 g of Pd/C (10% of Pd) were added and hydrogenation was carried out less than 2 bar. After filtration, the mixture was concentrated and the residue was taken in diethyl ether. The obtained crystals were filtered and dried, to give trandolapril
Yield = 42 gms
Melting point = 123-125 °C
Specific optical rotation = - 18.2° (C = 2 in ethanol)

### Example 8

### Preparation of Trandolapril

### (Using unresolved phenyl methyl octahydroindole-2-carboxylate hydrochloride)

Phenyl methyl octahydroindole-2-carboxylate hydrochloride 50gm (prepared in example 5) was taken in 500ml of water and 10gm of sodium hydroxide was added. The mass was stirred for one hour and 300ml of dichloromethane was added. The bottom organic layer was separated and aqueous layer discarded. The organic layer was dried over sodium sulphate. The dried organic layer was taken in a flask and ECPPA acid chloride hydrochloride, and triethylamine was added at room temperature. The mass was stirred for 3 hours at room temperature. The inorganic salt was filtered and the filtrate was concentrated under reduced pressure. The filtrate was concentrated in vacuo, the residue was taken up in 200ml of ethanol and 6.5 g of Pd/C (10% of Pd) were added and hydrogenation was carried out less than 2 bar. After filtration, the mixture was concentrated and the residue was taken in diethyl ether. The obtained crystals were filtered and dried to give trandolapril
Yield = 32 gms
Melting point = 123-125.8 °C
Specific optical rotation = - 18.1 °(C = 2 in ethanol)

## Claims

1. A process for the synthesis of a phenyl methyl-(2S, 3aR, 7aS)-octahydroindole-2-carboxylate hydrochloride of formula IX : 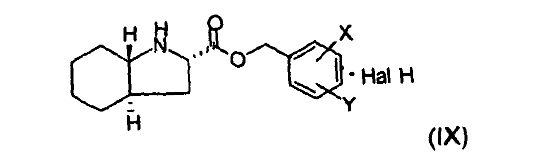 and its conversion to Trandolapril of formula I : 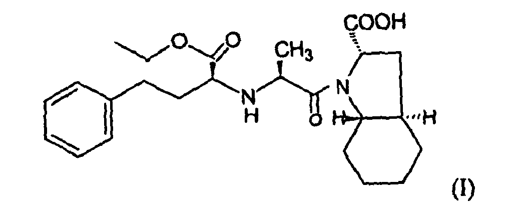 which comprises the steps of:
a) reacting methyl-β-hydroxy alaninate hydrochloride with an acylating agent in a non-polar solvent at 0-150°C to give a O,N-diacylated compound of formula II : 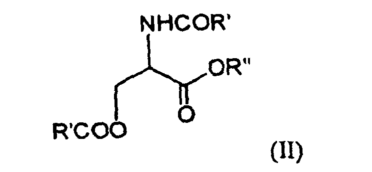
b) reacting the O,N-diacylated derivative of formula II with an enamine of cyclohexanone of formula IV : 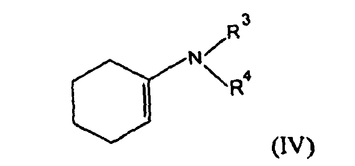 in the presence of an organic base, at 0-150°C to yield methyl-N-acyl-β-(2-oxo cyclohexyl) alaninate of formula V : 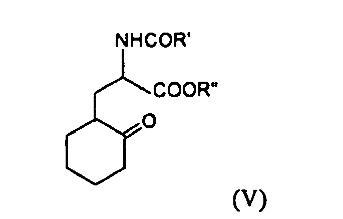
c) submitting said alaninate to a hydrolytic cyclization yielding an indole derivative of formula VI : 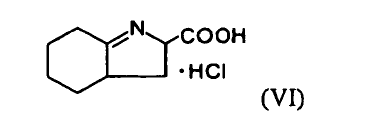
d) hydrogenating the indolic derivative of formula VI into a perhydro indole 2-carboxylic acid derivative of formula VII : 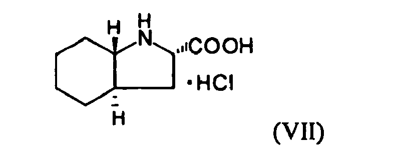
e) esterifying this perhydro indole carboxylic acid of formula VII with a benzyl alcohol of the formula VIIIa : 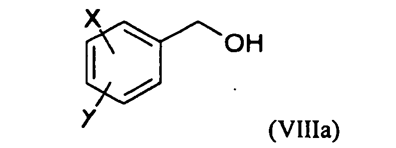 in the presence of an aryl sulfonic acid, to give the benzyl ester of formula VIII : 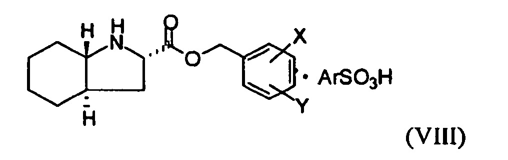 followed by conversion of the benzyl ester arylsulfonate of formula VIII to the hydrohalide of formula IX : 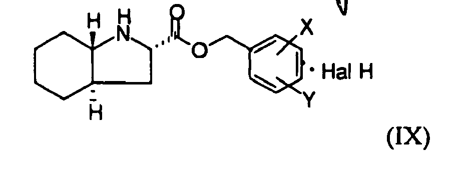
f) resolving and converting it into a benzyl (2S, 3aR, 7aS)-octahydroindole-2-carboxylate hydrohalide ;
g) coupling it with ECPPA acid chloride hydrochloride to get the ester of formula X and further debenzylating it by hydrogenolysis, to yield trandolapril of formula I.
X and Y are an hydrogen atom, an halogen, alkyl or alkoxy group and Ar is an aryl group.

2. A process as claimed in claim 1, wherein the acylation is achieved using a functional derivative of an alkylcarboxylic acid such as an anhydride, and the like.

3. A process as claimed in claim 1, wherein the enamine of cyclohexanone of formula IV derives from 1-pyrrolidine, 1-piperidine, 1-morpholine and the thus formed enamine is reacted with the product of formula II in a non polar solvent, at a temperature ranging from 0°C to 150°C.

4. A process as claimed in claim 1, wherein the product of formula V is cyclized with 1N to 10N hydrochloric acid at 0-100°C, to give an hexahydro indolic derivative of formula VI.

5. A process as claimed in claim 1 wherein the hydrogenation of the indolic derivative of formula VI is carried out in the presence of Pd/C, PtO₂/C, Rh/C or the like as acatalyst, in a acidic solvent like acetic acid or propanoic acid, at a temperature from 15°C to 50°C, using a hydrogen pressure ranging from 10 to 60 psi.

6. A process as claimed in claim 1, wherein the perhydrogenated product octahydroindole-2-carboxylic acid hydrochloride of formula VII is converted into its benzylester arylsulfonate and without being isolated, is further converted into the hydrohalide of formula IX.

7. A process as claimed in claim 1 wherein the hydrohalide of formula IX is resolved using an optically active organic acid selected among an aroyl L-tartaric acid, D-10 camphosulfonic acid, S-mandelic acid and the resulting salt is finally converted into phenyl methyl (2s, 3aR, 7aS)-octahydroindole-2-carboxylate hydrohalide of formula X :

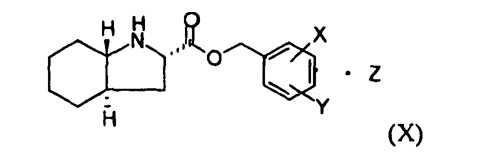

8. A process as claimed in claim 1, wherein the product of formula X is condensed with ECPPA acid chloride hydrochloride of formula XI : 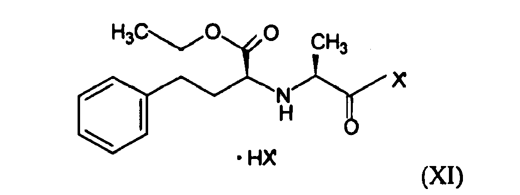 in the presence of a base, to give the product of formula XII : 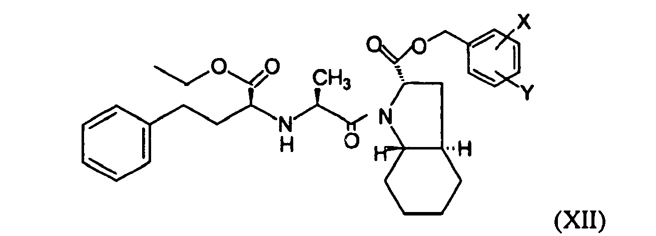 which on debenzylation, provides Trandolapril of formula I.

9. A process as claimed in claim 1, wherein the peptidic condensation is carried out in the presence of an organic base selected among triethylamine, disopropylethylamine, N-methylmorpholine.

10. A process as claimed in claim 1 wherein the debenzylation step is carried out using hydrogen and a catalyst selected among Pd/C, Rh/C, Pt/C and the isolated Trandolapril is purified using an organic solvent selected among ethanol and ethyl ether as the recrystallization solvent.

## Patentansprüche

1. Verfahren zur Synthese eines Phenylmetyl-(2S, 3aR, 7aS)-octahydroindol-2-carboxylat hydrochlorids der Formel IX : 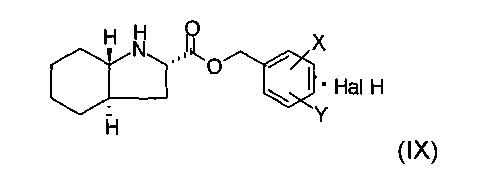 und dessen Konvertierung zu Trandolapril der Formel 1 : 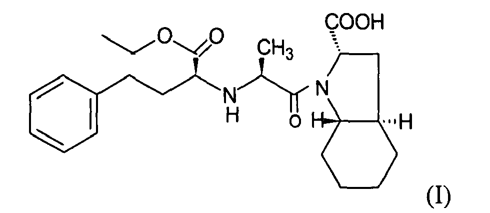 das folgende Schritte umfasst:
a) Reagieren von Methyl-β-hydroxy alaninat hydrochlorid mit einem Acylierungsmittel in einem nicht-polaren Lösemittel bei 0-150°C, um eine O,N-diacylierte Verbindung der Formel II zu ergeben: 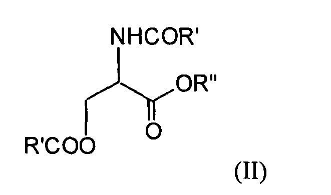
b) Reagieren des O,N-diacylierten Derivats der Formel II mit einem Enamin von Cyclohexanon der Formel IV: 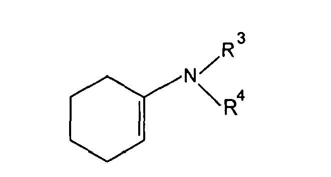 in Anwesenheit einer organischen Base bei 0-150°C, um Methyl-N-acyl-β-(2-oxocyclohexyl)-alaninat der Formel V zu ergeben: 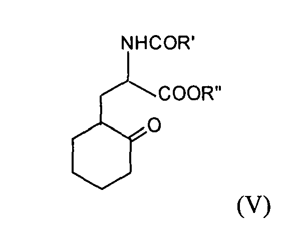
c) Das Alaninat einer hydrolytischen Cyclisierung unterziehln, die ein Indol-Derivat der Formel VI ergibt: 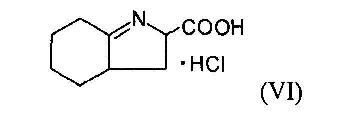
d) Hydrieren des Indol-Derivats der Formel VI in einem Perhydro-Indol-2-Carbonsäurederivat der Formel VII: 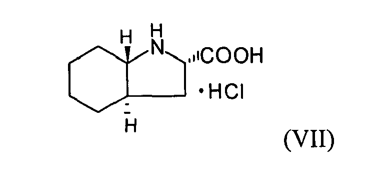
e) Verestern dieser Perhydro-Indol-Carbonsäure der Formel VII mit einem Benzylalkohol der Formel Villa: 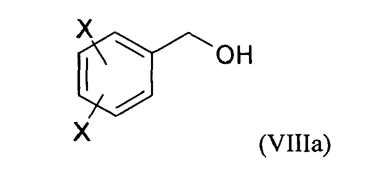 in Anwesenhenheit einer Arylsufonsäure, um den Benzylester der Formel VIII zu ergeben: 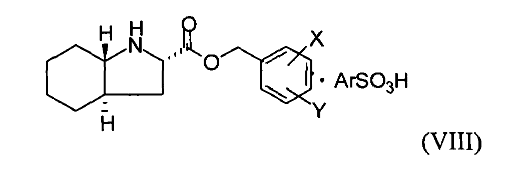 gefolgt von einer Konvertierung des Benzylester arylsulfonats der Formel VIII zum Hydrohalid der Formel IX: 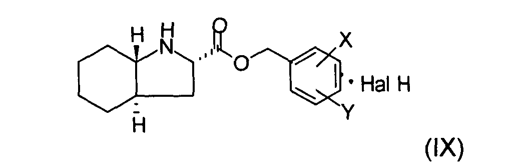
f) Lösen und Konvertieren desselben in ein Benzyl-(2S, 3aR, 7aS)-octahydroindol-2-carboxylatydrohalid.
g) Koppeln desselben mit ECPPA-Säurechloridhydrochlorid, um den Ester der Formel X zu erhalten und weiter Entbenzylieren desselben durch Hydrogenolyse, um das Transdolapril der Formel I zu ergeben.

2. Verfahren gemäß Anspruch 1, wobei die Acylierung unter Verwendung eines funktionalen Derivats einer Alkylcarbonsäure erreicht wird, wie beispielsweise eines Anhydrids und dergleichen.

3. Verfahren gemäß Anspruch 1, wobei das Enamin des Cyclohexanons der Formel IV von 1-Pyrrolidin, 1-Piperidin, 1-Morpholin abgeleitet ist und das auf diese Weise gebildete Enamin mit dem Produkt der Formel II in einem nicht-polaren Lösemittel bei einer Temperatur im Bereich von 0°C bis 150°C zur Reaktion gebracht wird.

4. Verfahren gemäß Anspruch 1, wobei das Produkt der Formel V mit 1N bis 10N Salzäure bei 0-100°C cyclisiert wird, um ein Hexahydroindolderivat der Formel VI zu ergeben.

5. Verfahren gemäß Anspruch 1, wobei die Hydrierung des Indolderivats der Formel VI in Anwesenheit von Pd/C, PtO₂/C, oder dergleichen als Katalysator in einem Säurelösemittel wie Essigsäure oder Propionsäure bei einer Temperatur von 15°C bis 50°C unter Anwendung eines Wasserstoffdrucks von 10 bis 60 psi ausgeführt wird.

6. Verfahren gemäß Anspruch 1, wobei das perhydrierte Produkt Octahydroindol-2-carbonsäure hydrochlorid der Formel VII in seinem Benzylesterarylsulfonat konvertiert und ohne isoliert zu werden weiter in das Hydrohalid der Formel IX konvertiert wird.

7. Verfahren gemäß Anspruch 1, wobei das Hydrohalid der Formel IX unter Anwendung einer optisch aktiven organischen Säure, ausgewählt aus einer Aroyl-L-Weinsäure, D-10-Camphosulfonäure, S-Mandelsäure gelöst wird und das resultierende Salz schließlich zu Phenylmethyl-(2S, 3aR, 7aS)-octahydroindol-2-carboxylat hydrohalid der Formel X konvertiert wird:

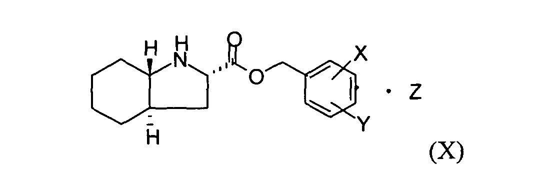

8. Verfahren gemäß Anspruch 1, wobei das Produkt der Formel X mit ECPPA-Säurechloridhydrochlorid der Formel XI: 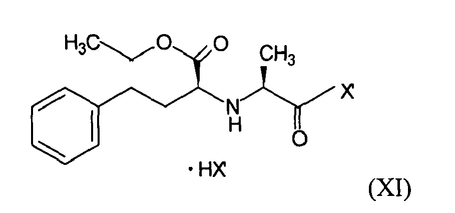 in Anwesenheit einer Base kondensiert wird, um das Produkt der Formel XXII zu ergeben: 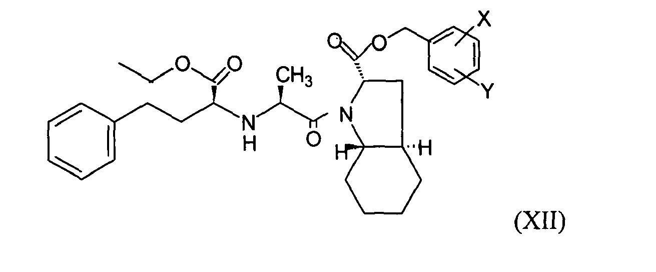 das nach Entbenzylierung, das Transdolapril der Formel I schafft.

9. Verfahren gemäß Anspruch 1, wobei die Peptid-Kondensation in Anwesenheit einer organischen Base ausgeführt wird, die aus Triethylamin, Disopropylethylamin, N-Methylmorpholin ausgewählt ist.

10. Verfahren gemäß Anspruch 1, wobei der Entbenzylierungsschritt unter Verwendung von Wasserstoff und einem Katalysator, ausgewählt aus Pd/C, Rh/C, Pt/C, ausgeführt wird und das isolierte Trandolapril unter Verwendung eines organischen Lösemittels gereinigt wird, ausgewählt aus Ethanol und Ethylether als Rekristallisierungs-Lösemittel.

## Revendications

1. Procédé de synthèse du chlorhydrate du (2S, 3aR, 7aS)- octahydro indole-2 carboxylate de phenylmethyle de formule IX 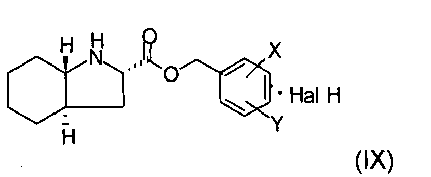 et de sa conversion en Trandolapril de formule I 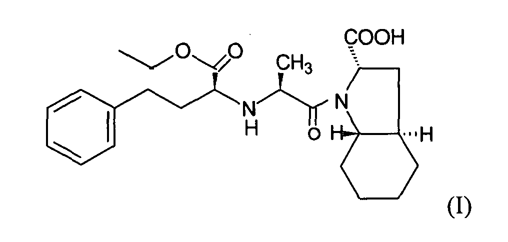 qui comprend les étapes de :
a - réaction du chlorhydrate de β-hydroxy alaninate de méthyle avec un agent d'acylation dans un solvant non polaire à 0-150°C pour fournir un composé O,N- diacylé de formule II 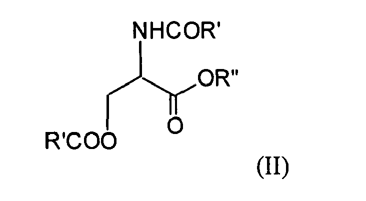
b - réaction du dérivé O,N- di acylé de formule II avec une cyclohexanone-enamine de formule IV 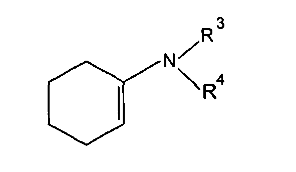 en présence d'une base organique à 0-150°C pour fournir un N-acyle β(2-oxocyclohexyl) alaninate de méthyle de formule V 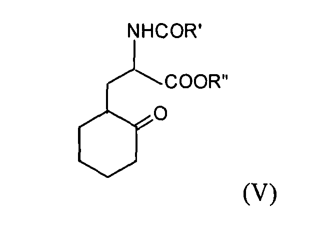
c - soumettre ledit alaninate à une cyclisation hydrolytique pour fournir un dérivé in dolique de formule VI 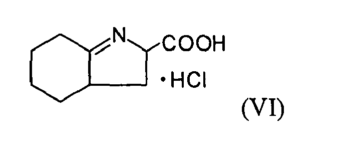
d - hydrogénation du dérivé in dolique de formule VI en un dérivé de l'acide per hydro indole carboxylique de formule VII 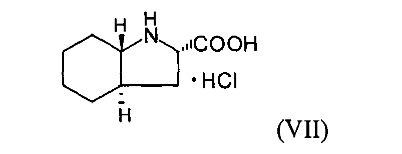
e - estérification de cet acide per hydro indole carboxylique de formule VII avec un alcool benzylique de formule Villa 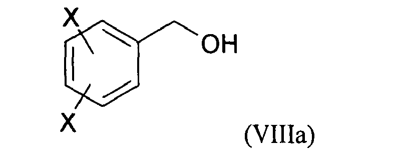 en présence d'un acide arylsulfonique pour donner l'ester benzylique de formule VIII 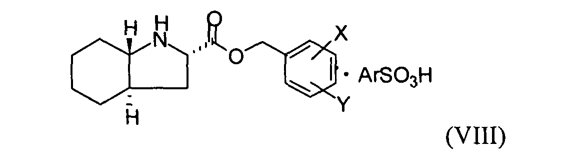 suivi de la conversion de l'arylsulfonate d'ester benzylique de formule VIII en halo hydrate de formule IX 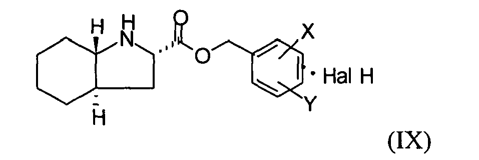
f - résolution et conversion en halo hydrate de (2S, 3aR, 7aS) octahydro 2-carboxylate de benzyle
g - couplage avec le chlorhydrate de chlorure d'acide d'ECPPA pour fournir l'ester de formule X et ensuite débenzylation par hydrogénolyse pour former le Trandolapril de formule (I).

2. Procédé selon la revendication 1, dans lequel l'acylation est effectuée en employant un dérivé fonctionnel d'acide alkyl carboxylique tel qu'un anhydride et similaire.

3. Procédé selon la revendication 1, dans lequel la cyclohexanone enamine de formule IV dérive de la 1-pyrrolidine, de la 1-pipéridine, de la 1-morpholine et dans lequel l'enamine ainsi formée est mise à réagir avec le composé de formule II dans un solvant non polaire à une température s'échelonnant de 0 à 150°C.

4. Procédé selon la revendication 1, dans lequel le composé de formule V est cyclisé avec l'acide chlorhydrique 1N à 10N, à 0 à 100°C pour fournir un dérivé hexahydro indolique de formule VI.

5. Procédé selon la revendication 1, dans lequel l'hydrogénation du dérivé indolique de formule VI est effectuée en présence de Pd/C, PtO₂/C, Rh/C ou similaire, en tant que catalyseur, dans un solvant acide tel que l'acide acétique ou l'acide propénoïque, à une température allant de 15°C à 50°C, en employant une pression d'hydrogène s'échelonnant de 10 à 60 psi.

6. Procédé selon la revendication 1, dans lequel le dérivé per hydrogéné - chlorhydrate de l'acide octahydroindole - 2 carboxylique - de formule d'arylsulfonate et sans être isolé, est ensuite converti en l'halo hydrate de formule IX.

7. Procédé selon la revendication 1, dans lequel l'halo hydrate de formule IX est dédoublé en employant un acide organique optiquement actif choisi parmi les acides aroyl L-Tartrique, l'acide D-10-Camphosulfonique et l'acide S-mandélique, et le sel en résultant est finalement converti en un halo hydrate de (2S, 3Ar, 7aS) octahydroindole - 2 carboxylate de phényle méthyle, de formule X.

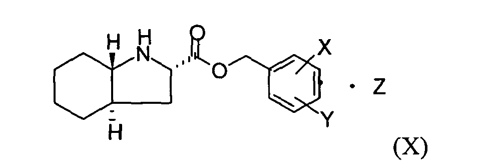

8. Procédé selon la revendication 1, dans lequel le composé de formule X est condensé avec le chlorhydrate de chlorure d'acide ECPPA de formule XI 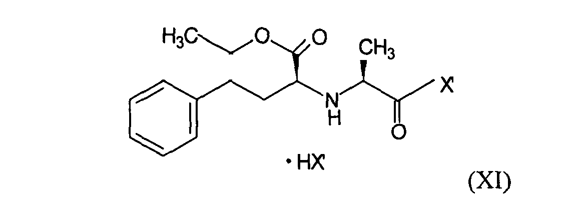 en présence d'une base pour fournir le composé de formule XII 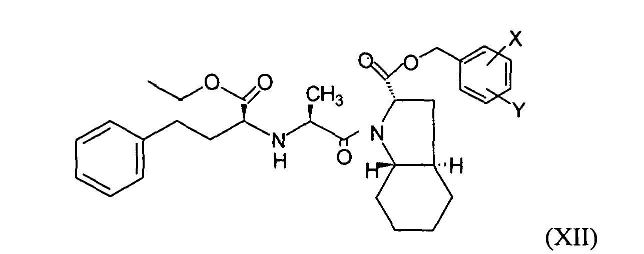 qui par débenzylation fournit le Trandolapril de formule I

9. Procédé selon la revendication 1, dans lequel la condensation peptidique est effectuée en présence d'une base organique choisie parmi la triméthylamine, la disopropyl éthylamine et la N-méthyl morphioline.

10. Procédé selon la revendication 1, dans lequel l'étape de débenzylation est effectuée en employant l'hydrogène et un catalyseur choisi parmi Pd/C, Rh/C et Pt/C et le Trandolapril isolé est purifié à l'aide d'un solvant organique choisi parmi l'éthanol et l'éther éthylique en tant que solvant de recristallisation.
